# EUROPÄISCHE PATENTANMELDUNG

(11) **EP 1 163 891 A1**
(43) Veröffentlichungstag der Anmeldung: **19.12.2001**
(21) Anmeldenummer: 01250220.9
(22) Anmeldetag: 15.06.2001
(51) Int. Cl.: A61F 2/30, A61F 2/34

(54) **Verschlusselement für Endoprothesensystem**

(30) Priorität: 17.06.2000 DE 10029637
(71) Anmelder: Biomet Merck Deutschland GmbH, 14167 Berlin (DE)
(72) Erfinder: Calisse, Jorge, 10785 Berlin (DE); Klas, Norbert, 64625 Bensheim (DE)
(74) Vertreter: Gross, Felix, Dr.

(57) **Zusammenfassung**

Endoprothesensystem umfassend ein Endoprothesenteil, insbesondere eine Hüftpfannenschale (1), zum Befestigen an einem Knochen des menschlichen oder tierischen Körpers mit wenigstens einer Durchgangsöffnung (3) zum Knochen und einem in einen Einsetzabschnitt (3.2) der Durchgangsöffnung (3) einsetzbaren Deckel (4) zum im wesentlichen vollständigen Verschließen der Durchgangsöffnung (3), wobei der Deckel (4) zum Erzeugen einer im eingesetzten Zustand quer zur Längsachse (3.1) der Durchgangsöffnung (3) wirkenden Verschlusskraft wenigstens abschnittsweise quer zur Längsachse (3.1) der Durchgangsöffnung (3) Übermaß gegenüber dem Einsetzabschnitt (3.2) der Durchgangsöffnung (3) aufweist und wobei der Deckel (4) als dünnes Plättchen ausgebildet ist, dessen Dicke derart gering und dessen Übermaß gegenüber dem Einsetzabschnitt (3.2) der Durchgangsöffnung (3) derart groß gewählt sind, dass der Deckel (4) beim unter Aufbringung einer zur Knochenseite gewandten Kraft erfolgenden Einsetzen in die Durchgangsöffnung (3) eine zur Knochenseite (1.2) hin gewölbte Gestalt annimmt.

## Beschreibung

Die vorliegende Erfindung betrifft ein Endoprothesensystem umfassend ein Endoprothesenteil, insbesondere eine Hüftpfannenschale, zum Befestigen an einem Knochen des menschlichen oder tierischen Körpers mit wenigstens einer Durchgangsöffnung zum Knochen und einem in einen Einsetzabschnitt der Durchgangsöffnung einsetzbaren Deckel zum im wesentlichen vollständigen Verschließen der Durchgangsöffnung, wobei der Deckel zum Erzeugen einer im eingesetzten Zustand quer zur Längsachse der Durchgangsöffnung Verschlusskraft wenigstens abschnittsweise quer zur Längsachse der Durchgangsöffnung Übermaß gegenüber dem Einsetzabschnitt der Durchgangsöffnung aufweist.

Endoprothesen im Sinne der vorliegenden Erfindung werden in der Regel eingesetzt, um beschädigte oder erkrankte Gelenke des menschlichen oder tierischen Körpers, wie beispielsweise das Hüftgelenk oder das Kniegelenk, zu ersetzen. Hierbei werden üblicherweise Teile des betroffenen Knochens entfernt und an diesen entsprechend vorbereiteten Stellen Teile der Endoprothese befestigt. So wird beispielsweise bei einer Hüftendoprothese ein Teil des Hüftknochens schalenförmig ausgefräst, um an dieser Stelle eine Hüftpfannenscha le einzusetzen und zu befestigen. Diese nimmt wiederum ein schalenförmiges Inlay auf, das meist aus Polyethylen besteht und als Widerlager für den kugelförmigen Gelenkkopf des Femurteils der Hüftgelenksprothese dient.

Die Verbindung zwischen dem am Knochen zu befestigenden Endoprothesenteil und dem Knochen kann bei diesen Endoprothesen auf vielfache verschiedene Weise realisiert sein. So können beispielsweise Knochenschrauben verwendet werden, die durch Durchgangsöffnungen im Endoprothesenteil hindurch mit dem Knochen verschraubt werden und so das Endoprothesenteil fixieren. Bei anderen Varianten weist das Endoprothesenteil selbst ein Gewinde auf, mit Hilfe dessen es in den Knochen eingeschraubt werden kann. Weiterhin ist es möglich, das Endoprothesenteil durch so genannten Knochenzement zu fixieren. Ebenso ist es möglich, einen Presssitz zwischen Knochen und Endoprothesenteil vorzusehen. Die einzelnen Varianten der Fixierung können gegebenenfalls auch miteinander kombiniert werden.

Unabhängig von der Art der Fixierung weisen viele dieser Endoprothesenteile Durchgangsöffnungen zum Knochen hin auf. Diese dienen entweder, wie oben erwähnt, zur Aufnahme von Knochenschrauben und/oder zur Aufnahme von Teilen eines Einsetzwerkzeugs, mit dem das Endoprothesenteil in den Knochen eingesetzt bzw. auf diesen aufgesetzt wird. Diese Durchgangsöffnungen bringen das Problem mit sich, dass durch sie hindurch Verschleißpartikel des künstlichen Gelenks, insbesondere Polyethylenpartikel, an den Knochen gelangen können. Solche Verschleißpartikel setzen bei entsprechend geringer Größe Körperabwehrreaktionen in Gang, die auch den umliegenden Knochen in Mitleidenschaft ziehen. Dies kann bis hin zum Lockern der Endoprothese führen.

Um diesem Problem zu begegnen, wurden Endoprothesensysteme vorgeschlagen, bei denen solche Durchgangsöffnungen zum Knochen mit einem Deckel verschlossen werden. Ein solches gattungsgemäßes Endoprothesensystem ist beispielsweise aus dem US Patent Nr. 5,725,580 bekannt. Bei diesem wird ein relativ dicker Deckel in die Durchgangsöffnung zum Verschließen derselben eingeführt. Der ansonsten flache Deckel weist einen sich senkrecht zur Deckelebene erstreckenden, umlaufenden Deckelrand auf, der Übermaß gegenüber der Durchgangsöffnung hat. Er wird mit dem Rand voran in die Durchgangsöffnung eingeführt, so dass er sich infolge des Übermaßes des Deckelrandes gegenüber der Durchgangsöffnung elastisch von der Knochenseite weg wölbt. Die elastische Verformung des Deckels bewirkt eine entsprechende Verschlusskraft zwischen Deckel und Endoprothesenteil.

Dieses bekannte Endoprothesensystem weist zum einen den Nachteil auf, dass der Deckel relativ aufwändig gestaltet und daher in seiner Herstellung vergleichsweise teuer ist.

Nachteilig ist zudem, dass der Deckel mit dem Rand voraus in die Durchgangsöffnung eingeführt werden muss. Hierbei kommt es leicht zum Verkippen des Deckels bezüglich der Achse der Durchgangsöffnung, woran auch die Fase am vorlaufenden Deckelrand nichts ändert. Es ist daher erforderlich, den Deckel mit einem entsprechenden Werkzeug oder dergleichen zu halten und in die Durchgangsöffnung einzuführen, wobei allerdings besondere Sorgfalt aufzuwenden ist. Zudem ist die Gefahr relativ hoch, dass der Deckel schief eingesetzt wird und damit zum einen nicht nur seine Verschlussfunktion nicht erfüllt, sondern unter Umständen sogar bereichsweise über die Kontur des Endoprothesenteils hinausragt und somit selbst die Ursache für schädlichen Abrieb am eingesetzten Polyethylen-Inlay ist.

Ein weiterer Nachteil dieses Endoprothesensystems liegt darin, dass die Verriegelung des Deckels in der Durchgangsöffnung gegenüber Kräften, die von Knochenseite her auf den Deckel wirken, nur in sehr geringem Maße wirksam ist. So kann beispielsweise Knochenzement beim Einsetzen der Endoprothesenteils in die Durchgangsöffnung eindringen und von der Knochenseite her auf den von der Knochenseite weg gewölbten Deckel eine die Verriegelungskraft vermindernde Lösekraft ausüben. Dies kann zum einen zum Lösen des Deckels führen. Noch nachteiliger wirkt es sich jedoch aus, wenn lediglich eine unbemerkte Lockerung des Deckels erfolgt und dieser sich erst im Laufe der Zeit löst, da er dann ebenfalls nicht nur seine Verschlussfunktion nicht erfüllt, sondern auch wiederum selbst Quelle von schädlichem Abrieb sein kann.

Der vorliegenden Erfindung liegt daher die Aufgabe zu Grunde, ein gattungsgemäßes Endoprothesensystem zur Verfügung zu stellen, das bei einfacher Herstellbarkeit und Handhabung einen einfachen und zuverlässigen Verschluss der Durchgangsöffnung im Endoprothesenteil sicherstellt.

Diese Aufgabe wird ausgehend von einem Endoprothesensystem gemäß dem Oberbegriff des Anspruchs 1 durch die im kennzeichnenden Teil des Anspruchs 1 angegebenen Merkmale gelöst.

Der Erfindung liegt die technische Lehre zu Grunde, dass man einen besonders einfach herzustellenden und zuverlässigen Verschluss der Durchgangsöffnung erhält, wenn der Deckel als dünnes Plättchen ausgebildet ist, dessen Dicke derart gering und dessen Übermaß gegenüber der Durchgangsöffnung derart groß gewählt sind, dass der Deckel beim Einsetzen in die Durchgangsöffnung, welches unter Aufbringung einer zur Knochenseite gewandten Kraft erfolgt, eine zur Knochenseite hin gewölbte Gestalt annimmt.

Ein Vorteil der erfindungsgemäßen Lösung liegt in der Konfiguration des Deckels im eingesetzten Zustand. Zum einen ist durch die sich ergebende kuppelartige Gestalt des Deckels sichergestellt, dass eine ausreichende Verschlusskraft wirkt, welche den Deckel in seiner Position hält. Die Wölbung zur Knochenseite hin bewirkt zum anderen, dass sich die zwischen dem Deckelrand und der Wandung der Durchgangsöffnung ergebenden Verschlusskraft beim Auftreten einer Lösekraft von der Knochenseite her, wie sie beispielsweise durch in die Durchgangsöffnung eindringenden Knochenzement beim Einsetzen des Endoprothesenteils oder durch andere einen entsprechenden Druck ausübende Medien auftreten kann, noch weiter erhöht und somit einem Lockern oder gar Lösen des Deckels entgegenwirkt.

Durch die gewählte Gestaltung ist weiterhin ein einfaches Einsetzen des Deckels in die Durchgangsöffnung gewährleistet. Der Chirurg muss lediglich den Deckel in Richtung der Knochenseite drücken. Dieser nimmt hierbei auf Grund des Übermaßes gegenüber der Durchgangsöffnung und seiner geringen Dicke schon bei Aufbringen einer geringen Kraft eine zur Knochenseite hin gewölbte Gestalt an. Durch diese ist wiederum sichergestellt, dass das Plättchen beim weiteren Eindrücken in die Durchgangsöffnung nicht verkippt. Es sind keine besonderen Einsetzwerkzeuge erforderlich. Das Einsetzen kann einfach unter Zuhilfenahme eines Fingers oder dergleichen erfolgen.

Ein zusätzlicher Vorteil der erfindungsgemäßen Gestaltung liegt in der sehr einfachen Gestaltung des Deckels als dünnes Plättchen. Hierbei kann es sich um ein einfaches Stanzteil handeln. Ein aufwändiges Schneiden oder eine sonstwie aufwändige Bearbeitung ist nicht erforderlich.

Der Deckel kann als im Ausgangszustand im wesentlichen flaches Plättchen ausgebildet sein. Hierdurch läßt er sich besonders schnell und einfach herstellen, beispielsweise einfach aus einem entsprechenden ebenen Halbzeug ausstanzen, ausschneiden etc. Er kann aber natürlich auch in der entsprechenden Form gegossen sein.

Vorzugsweise ist der Deckel als im Ausgangszustand gewölbtes Plättchen ausgebildet. Hierdurch ergibt sich zum einen eine automatische Zentrierung des Deckels beim Einsetzen in die Durchgangsöffnung. Zum anderen ist das Einsetzen des Deckels noch weiter erleichtert, da er schon in der gewünschten Weise vorverformt ist und die weitere Verformung in dieser Richtung daher einfacher von Statten geht. Auch diese Ausführungen lassen sich einfach herstellen, da beispielsweise dem Ausstanzen oder Ausschneiden des Deckels lediglich noch ein kurzer Tiefziehvorgang oder dergleichen folgen muss.

Die Form des Deckels ist bevorzugt an die Kontur der Durchgangsöffnung angepasst, so dass ein im wesentlichen vollständiges Verschließen der Durchgangsöffnung sichergestellt ist. Übermaß und Dicke des Deckels sind also vorzugsweise so gewählt, dass sich der Deckel beim Einsetzen in die Durchgangsöffnung so verformt, dass der Deckelrand im Wesentlichen vollständig an der Wandung der Durchgangsöffnung anliegt. So kann die Gestaltung des Deckels für eine kreisrunde Durchgangsöffnung beispielsweise so gewählt sein, dass er im Ausgangszustand einen ebenfalls kreisrunden Deckelrand aufweist und sich beim Einsetzen in die Durchgangsöffnung eine über den Umfang im wesentlichen gleichbleibende Wölbung des Deckels ergibt, der Deckel also mithin in jedem Axialschnitt im wesentlichen dieselbe gewölbte Schnittkontur aufweist.

Der Deckel kann aber auch in seiner Ausgangskonfiguration so gewählt sein, dass sich beim Einsetzen in die Durchgangsöffnung - vorzugsweise radial gerichtete-Sicken, Rippen oder dergleichen ergeben, welche den domartig gewölbten Deckel noch weiter stabilisieren. Der Deckel kann hierzu in seinem Ausgangszustand bereits vorverformt sein, beispielsweise bereits mit entsprechenden Sicken versehen sein, die sich beim Einsetzen dann noch deutlicher ausbilden. Der Deckelrand ist dann wiederum vorzugsweise so gestaltet, dass er im eingesetzten Zustand auch im Bereich der Sicken an der Wandung der Durchgangsöffnung anliegt. Dies läßt sich beispielsweise dadurch erzielen, dass der Deckel im Bereich dieser Sicken etwas größeres Übermaß gegenüber der Durchgangsöffnung aufweist als im übrigen Bereich. Eine solche Gestaltung mit über den Umfang variierendem Übermaß kann auch ausreichen, um beim Einsetzen des Deckels die Ausbildung solcher Sicken zu bewirken, ohne dass der Deckel im Ausgangszustand bereits entsprechend vorverformt ist.

Bei bevorzugten Varianten des erfindungsgemäßen Endoprothesensystems sind die Dicke des Deckels derart gering und das Übermaß des Deckels gegenüber der Durchgangsöffnung derart groß gewählt, dass er beim Einsetzen in die Durchgangsöffnung plastisch verformt wird. Hierdurch ergibt sich in vorteilhafter Weise eine Verfestigung des Deckels in dieser Konfiguration im eingesetzten Zustand.

Weitere günstige Ausführungsformen des erfindungsgemäßen Endoprothesensystems zeichnen sich dadurch aus, dass die Dicke und/oder das Übermaß des Deckels gegenüber der Durchgangsöffnung zum Erzielen einer Verschlusskraft gewählt sind, bei der das Endoprothesenteil im Kontaktbereich mit dem in den Einsetzbereich eingesetzten Deckel zumindest abschnittsweise plastisch verformt wird. Der Deckel drückt bei diesen Varianten mit anderen Worten je nach Gestaltung mit seinem Rand selbst eine oder mehrere Nuten in die Wandung der Durchgangsöffnung, in die sein Rand dann eingreift. Der Deckel verriegelt sich hierbei sozusagen selbst in seiner eingesetzten Position. Es versteht sich, dass insbesondere bei diesen Varianten die Werkstoffpaarung Endoprothesenteil - Deckel entscheidenden Einfluss auf die Gestaltung des Deckels hat.

Bei weiteren Varianten des erfindungsgemäßen Endoprothesensystems sind zur Verbesserung des Sitzes des Deckels im Einsetzabschnitt Rastmittel zum Einrasten des Deckelrandes in der Durchgangsöffnung vorgesehen. Diese können von entsprechenden Vorsprüngen in der Durchgangsöffnung gebildet sein. Einfacher und kostengünstiger lassen sich jedoch Rastmittel in Form von Hinterschneidungen der Durchgangsöffnung herstellen. So können beispielsweise eine oder mehrere, vollständig oder mit Unterbrechungen umlaufende Nuten oder dergleichen vorgesehen sein.

Besonders einfach und kostengünstig herzustellende Varianten ergeben sich, wenn die Rastmittel von einem Gewinde im Einsetzabschnitt der Durchgangsöffnung gebildet sind. Dieses ist aus Gründen der weiteren Vereinfachung vorzugsweise als Standardgewinde, insbesondere als metrisches Gewinde ausgebildet. Ein weiterer Vorteil ergibt sich, wenn dieses Gewinde mit abgestumpften Gewindezähnen ausgebildet ist, also beispielsweise ein Standardgewinde mit verringerter Gewindetragtiefe ist. Durch die stumpfe Ausgestaltung der Gewindezähne wird dabei verhindert, dass beim Einsetzen des Deckels vom Deckel oder dem Endoprothesenteil Späne oder dergleichen abscheren, die dann als schädliche Verunreinigung an den Knochen oder in den Bereich der Gelenkpaarung gelangen, wo sie den Verschleiß der Reibpartner beschleunigen.

Besonders guten Sitz des Deckels und einfaches Einführen ohne die Gefahr von Verkantungen des Deckels erzielt man, wenn das Gewinde möglichst geringe Steigung aufweist. Hierzu kann es beispielsweise als Standardgewinde, insbesondere als metrisches Gewinde, mit verringerter Steigung ausgebildet sein. Ein vergleichbarer Effekt wird erzielt, wenn das Gewinde mehrgängig ausgebildet ist.

Bei allen Varianten mit einem Gewinde als Rastmittel in der Durchgangsöffnung ist der Deckel wenigstens einem Abschnitt in seinem Randbereich vorzugsweise so dünn und damit einfach verformbar ausgebildet, dass er zum einen der Verformung am Übergang zwischen zwei in Achsrichtung des Gewindes benachbarten Abschnitten der Gewinderille (bei einem eingängigen Gewinde) bzw. am Übergang zwischen zwei in Achsrichtung des Gewindes benachbarten Gewinderillen (bei einem mehrgängigen Gewinde) keinen erheblichen Widerstand entgegensetzt und sich zum anderen so an den dabei zu überwindenden Gewindezahn anschmiegt, dass ein zuverlässiger Verschluss sichergestellt ist.

Die vorliegende Erfindung läßt sich bei beliebigen Endoprothesensytemen, beispielsweise bei Knieendoprothesen, Ellenbogenendoprothesen etc. einsetzen. Besonders vorteilhaft läßt sie sich bei Endoprothesensystemen im Hüftbereich anwenden, da deren entsprechende Endoprothesenteile meist relativ große zu verschließende Durchgangsöffnungen zum Knochen hin aufweisen. Das Endoprothesenteil ist in diesem Fall eine Hüftpfannenschale, die in den Hüftknochen eingesetzt wird und wiederum ein Inlay aufnimmt, das als Reibpartner für den Kugelkopf des Femurteils der Endoprothese dient.

Die vorliegende Erfindung betrifft weiterhin ein Endoprothesenteil für ein erfindungsgemäßes Endoprothesensystem mit einer Durchgangsöffnung zum Knochen, die einen Einsetzbereich für einen Deckel zum Verschließen der Durchgangsöffnung mit Rastmitteln zum Einrasten des Deckelrandes aufweist, wobei die Rastmittel zum Einrasten des Deckelrandes in der oben beschriebenen vorteilhaften Weise von einem Gewinde gebildet sind.

Die Erfindung betrifft weiterhin einen Deckel zum Verschließen einer Durchgangsöffnung in einem Endoprothesenteil eines erfindungsgemäßen Endoprothesensystems, der in der oben beschriebenen vorteilhaften Weise als dünnes gewölbtes Plättchen ausgebildet ist.

Weitere bevorzugte Ausgestaltungen der vorliegenden Erfindung ergeben sich aus den Unteransprüchen und der nachstehenden Beschreibung bevorzugter Ausführungen der Erfindung unter Bezugnahme auf die beigefügten Zeichnungen. Es zeigen:
- Figur 1: einen Axialschnitt durch ein bevorzugtes Ausführungsbeispiel der vorliegenden Erfindung;
- Figur 2: eine Detailansicht der Ausführung aus Figur 1;
- Figur 3: eine Detailansicht einer weiteren bevorzugten Ausführungsform der vorliegenden Erfindung;
- Figur 4a bis 4c: Ansichten eines Deckels für ein erfindungsgemäßes Endoprothesensystem.

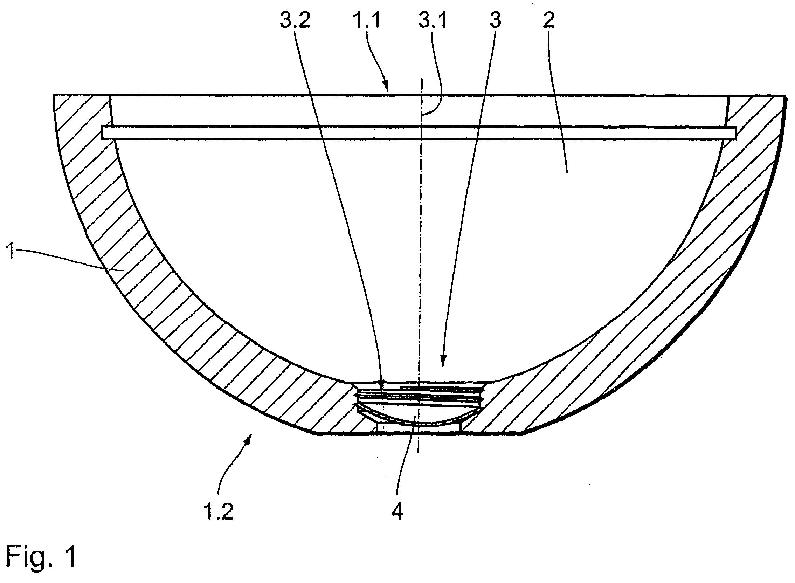

Figur 1 zeigt einen Axialschnitt durch eine bevorzugte Ausführung eines Teils eines erfindungsgemäßen Endoprothesensystems. Es handelt sich hierbei um eine Hüfteendoprothese mit einer Hüftpfannenschale 1. Diese Hüftpfannenschale 1 weist eine dem Gelenk zugewandte Gelenkseite 1.1 und eine den Knochen zugewandte Knochenseite 1.2 auf. Auf der Gelenkseite 1.1 befindet sich eine Ausnehmung 2, die zur Aufnahme eines in Figur 1 nicht dargestellten schalenförmigen Polyethylen-- Inlays dient, welches wiederum das Widerlager für den Kugelkopf des Femurteils der Hüfteendoprothese bildet.

Am Grund der Ausnehmung 2 ist eine Durchgangsöffnung 3 zur Knochenseite 1.2 hin angeordnet, deren Längsachse 3.1 mit der Symmetrieachse der Hüftpfannenschale 1 zusammenfällt. In diese Durchgangsöffnung 3 ist im Bereich eines Einsetzabschnitts 3.2 ein Deckel 4 eingesetzt, der die Durchgangsöffnung 3 im eingesetzten Zustand vollständig verschließt.

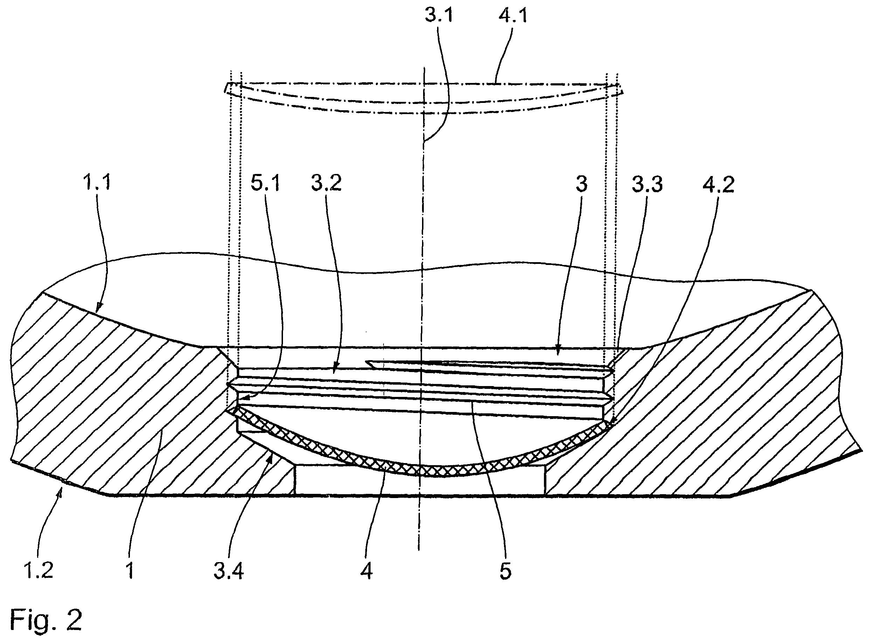

Figur 2 zeigt als Detail der Ansicht aus Figur 1 eine vergrößerte Darstellung im Bereich der Durchgangsöffnung 3. In diese Durchgangsöffnung 3 ist der Deckel 4 eingesetzt, der im Ausgangszustand Übermaß gegenüber dem Einsetzabschnitt 3.2 der Durchgangsöffnung 3 aufweist, wie in Figur 2 durch die gestrichelte Kontur 4.1 angedeutet ist. Wie anhand der Kontur 4.1 ebenfalls ersichtlich ist, weist der Deckel 4 im Ausgangszustand bereits eine leicht gewölbte Gestalt auf. Hierdurch erleichtert sich das Einsetzen des Deckels in die Durchgangsöffnung. Eine zusätzliche Hilfe beim Einsetzen bildet im Übrigen die Fase 3.3 am gelenkseitigen Ende der Durchgangsöffnung 3.

Es versteht sich, dass der Deckel bei anderen Ausführungen auch als einfaches, flaches Plättchen ohne entsprechende Vorverformung ausgebildet sein kann. Hierbei kann schon eine entsprechende Fase der Durchgangsöffnung ausreichen, um ein einfaches Einsetzen des Deckels zu gewährleisten. Eine solche Fase ist jedoch in keinem Fall zwingend erforderlich. Sie erleichtert wie erwähnt lediglich das Einsetzen des Deckels in die Durchgangsöffnung. Hierbei ist sie vorzugsweise so dimensioniert, dass ihre größte Querabmessung quer zur Längsachse der Durchgangsöffnung die Querabmessung des Deckels im Ausgangszustand übersteigt. Hierdurch ist ein besonders einfaches und glattes Einsetzen des Deckels sichergestellt, da eine solche Fase den gewünschten Verformungsvorgang des Deckels in vorteilhafter Weise unterstützt.

Der Deckel 4 wurde im gezeigten Beispiel unter Aufbringung einer zur Knochenseite 1.2 gewandten Kraft von der Gelenkseite 1.1 her in den Einsetzabschnitt 3.2 der Durchgangsöffnung 3 eingedrückt. Hierbei wurde er infolge seines quer zur Längsachse 3.1 der Durchgangsöffnung 3 bestehenden Übermaßes gegenüber dem Einsetzabschnitt 3.2 der Durchgangsöffnung 3 so verformt, dass er im eingesetzten Zustand eine zur Knochenseite 1.2 hin gewölbte Gestalt aufweist. Die Wölbung ist dabei stärker als im Ausgangszustand des Deckels 4. Das Übermaß des Deckels 4 gegenüber der Durchgangsöffnung 3 und die Dicke des Deckels 4 sind so gewählt, dass der Deckel 4 mit seinen Deckelrand im eingesetzten Zustand im wesentlichen vollständig an der Wandung der Durchgangsöffnung 3 anliegt. Übermaß und Dicke des Deckels 4 sind mit anderen Worten so gewählt, dass dessen Verformung beim Einsetzen über seinen Umfang im wesentlichen gleichmäßig, d. h. ohne unerwünschtes lokales Auswellen oder Einknicken erfolgt.

Auf Grund der Verformung beim Einsetzen in den Einsetzabschnitt 3.2 der Durchgangsöffnung 3 übt der Deckel 4 infolge einer aus dem elastischen Verformungsanteil resultierenden Rückstellkraft im eingesetzten Zustand eine quer zur Längsachse 3.1 der Durchgangsöffnung 3 gerichtete Verschlusskraft auf die Wandung des Einsetzabschnitts 3.2 aus. Diese Verschlusskraft hält den Deckel 4 in seiner Position in der Durchgangsöffnung 3.

Im gezeigten Beispiel sind im Bereich des Einsetzabschnitts 3.2 Rastmittel 5 angeordnet, in die der Deckelrand 4.2 des Deckels 4 infolge der im eingesetzten Zustand bestehen elastischen Verformung und der daraus resultierenden Rückstellkraft zu seiner Verriegelung im Einsetzabschnitt 3.2 eingreift, d. h. einrastet. Diese Rastmittel sind von einem eingängigen Gewinde gebildet. Es handelt sich hierbei um ein metrisches Gewinde mit verringerter Gewindetragtiefe und verringerter Steigung. Im gezeigten Beispiel handelt es sich um ein metrisches Gewinde M16×0,35, bei dem der Bohrungsdurchmesser 15,8 mm an Stelle der bei normaler Gewindetragtiefe vorgesehenen 15,62 mm beträgt. Es versteht sich jedoch, dass bei anderen Ausführungen auch entsprechend modifizierte Gewinde mit anderen Nennabmessungen verwendet werden können. Selbstverständlich können auch mehrgängige Gewinde verwendet werden.

Die verringerte Steigung des Gewindes 5 reduziert das Risiko, dass der Deckel 4 beim Einsetzen in den Einsetzbereich 3.2 der Durchgangsöffnung 3 verkippt und damit nicht richtig eingesetzt wird. Infolge der verringerten Gewindetragtiefe ergeben sich abgestumpfte Gewindezähne 5.1. Diese Abstumpfung der Gewindezähne verhindert, dass beim Einsetzen des Deckels 4 in die Durchgangsöffnung 3 vom Deckel 4 oder vom Gewinde 5 Späne oder dergleichen abgeschert bzw. abgelöst werden, die dann an den Knochen oder in den Bereich der Gelenkpaarung gelangen könnten und dort in bekannter Weise entsprechenden Schaden anrichten könnten. Ein weiterer Vorteil der abgestumpften Gewindezähne 5.1 liegt darin, dass der Deckelrand 4.2 an der Stelle, an der er über den Gewindezahn hinweg verläuft, um vom Eingriff in einen Abschnitt der Gewinderille in den in Achsrichtung benachbarten Abschnitt der Gewinderille zu wechseln, im Vergleich zum herkömmlichen Gewinde ein kleineres Hindernis in Form des abgestumpften Gewindezahnes 5.1 zu überwinden hat.

Der Deckel 4 ist im gezeigten Beispiel ein dünnes, kreisrundes, im Ausgangszustand bereits leicht gewölbtes metallisches Plättchen, dessen Dicke bei 0,08 mm liegt. Sein Durchmesser beträgt im Ausgangszustand 17 mm. Durch diese Dimensionierung ist sichergestellt, dass sich das Plättchen beim Einführen in die Durchgangsöffnung in der gewünschten Weise unter Einschluss eines plastischen Verformungsanteils verformt. Es versteht sich jedoch, dass insbesondere in Abhängigkeit von der Größe und Gestalt der Durchgangsöffnung auch andere Abmessungen und Plättchenkonturen gewählt werden können.

Es versteht sich weiterhin, dass an Stelle des Gewindes auch andere Rastmittel vorgesehen sein können. So kann beispielsweise eine durchgehend oder abschnittsweise umtaufende Nut im Bereich des Einsetzabschnitts angeordnet sein.

Durch die gewählte Konfiguration des Deckels 4 ergibt sich ein sicherer Verschluss der Durchgangsöffnung 3. Die Wölbung des Deckels 4 zur Knochenseite 1.2 hin bewirkt, dass sich beim Auftreten einer von der Knochenseite 1.2 her auf den Deckel 4 wirkenden Lösekraft die Verschlusskraft und somit auch die Verriegelung des Deckels im Gewinde 5 noch weiter erhöht. Ein Absatz 3.4 in der Durchgangsöffnung 3 verhindert im Übrigen das Lösen des Deckels 4 aus der Durchgangsöffnung 3 in Richtung der Knochenseite 1.2.

Zum Lösen des Deckels 4 aus der Durchgangsöffnung 3 muss dieser bei der in Figur 1 und 2 gezeigten Konfiguration sozusagen "zerstört" werden. Dies kann einfach dadurch geschehen, dass von der Knochenseite her eine entsprechend hohe Kraft aufgebracht wird, die den Deckel dann so stark verformt, dass er sich aus seinem Eingriff mit dem Gewinde löst.

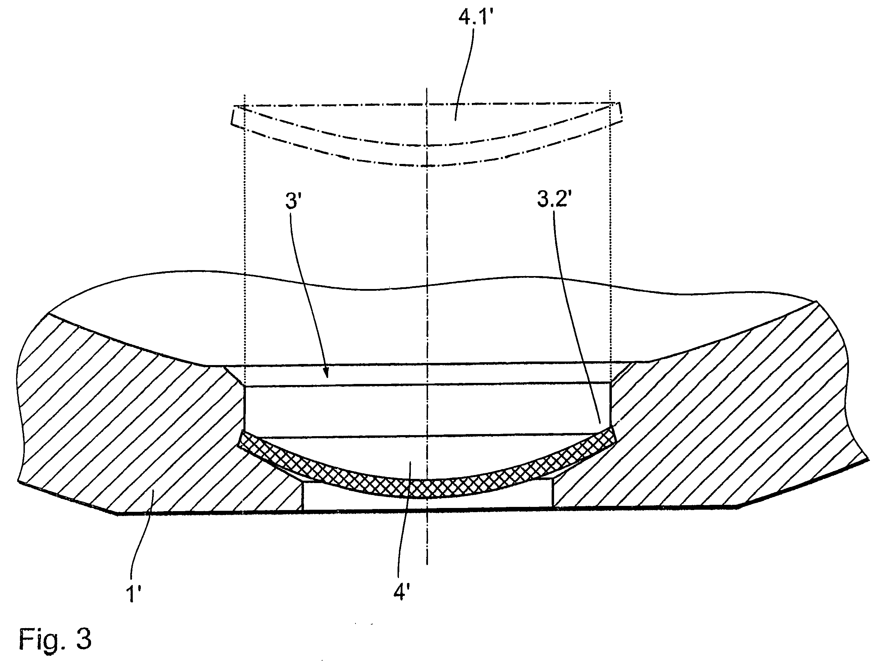

Figur 3 zeigt ein anderes Ausführungsbeispiel des erfindungsgemäßen Endoprothesensystems. Dieses gleicht in seinem grundsätzlichen Aufbau demjenigen aus Figur 1, so dass hier lediglich auf die Unterschiede eingegangen werden soll.

Der wesentliche Unterschied liegt zum einen darin, dass im Bereich des Einsetzabschnitts 3.2' der Durchgangsöffnung 3' in der Hüftpfannenschale 1' kein Gewinde vorgesehen ist, sondern der Einsetzabschnitt 3.2' von einer einfachen zylindrischen Bohrung gebildet ist. Ein weiter wesentlicher Unterschied liegt in der Gestaltung des Deckels 4'. Dieser ist zum einen dicker als der in Figur 1 gezeigte Deckel, zum anderen weist er, wie durch die gestrichelte Kontur 4.1' angedeutet ist, schon im Ausgangszustand eine stärkere Wölbung als der Deckel aus Figur 1 auf. Sein Übermaß gegenüber dem Einsetzabschnitt 3.2' der Durchgangsöffnung 3' ist geringer als bei der Ausführung aus Figur 1.

Durch die gewählte Gestaltung des Deckels 4' wird erreicht, dass er im in die Durchgangsöffnung 3' eingesetzten Zustand eine Verschlusskraft auf die Hüftpfannenschale 1' ausübt, die so groß ist, dass die Hüftpfannenschale 1' im Kontaktbereich mit den Deckel 4' plastisch verformt wird. Der Deckel 4' drückt sich infolge der im eingesetzten Zustand wirkenden elastischen Rückstellkraft mit anderen Worten selbst eine umlaufende Nut in die Wandung der Durchgangsöffnung 3', die dann neben der Verschlusskraft als zusätzliche Verriegelung des Deckels 4' in dieser Position dient. Je nachdem, ob der Deckel 4' in Umfangrichtung ein gleich bleibendes Übermaß gegenüber der Durchgangsöffnung 3' aufweist oder abschnittsweise ein größeres Übermaß als im übrigen Umfangsbereich aufweist, läuft die sich ergebende Nut nur abschnittsweise um bzw. läuft sie durchgehend mit gleich bleibender oder variierender Tiefe um. Es versteht sich im Übrigen, dass der Deckei auch bei den anderen beschriebenen Varianten mit abschnittsweise unterschiedlichem Übermaß gegenüber dem Einsetzabschnitt der Durchgangsöffnung ausgebildet sein kann.

Die stärkere Wölbung bzw. das geringere Übermaß des Deckels 4' gegenüber der Durchgangsöffnung 3' bewirkt, dass der Deckel 4' trotz der größeren Dicke bequem in den Einsetzabschnitt 3.2' eingesetzt werden kann. Die größere Dicke des Deckels 4' sorgt für eine entsprechend große Verschlusskraft, die ausreicht, um die Hüftpfannenschale 1' in der beschriebenen Weise plastisch zu verformen. Die plastische Verformung der Hüftpfannenschale 1' tritt beim Einsetzen erst dann ein, wenn die Kraft zum Einsetzen des Deckels 4' nicht mehr auf diesen wirkt, d. h. wenn die volle aus dem elastischen Anteil der Deckelverformung resultierende Rückstell- bzw. Verschlusskraft auf die Wandung der Durchgangsöffnung 3' wirkt. Es versteht sich, dass der Deckel bei anderen entsprechenden Ausführungen aber auch nur rein elastisch verformt werden kann. Ebenso kann diese Variante natürlich auch mit entsprechenden Rastmitteln kombiniert werden.

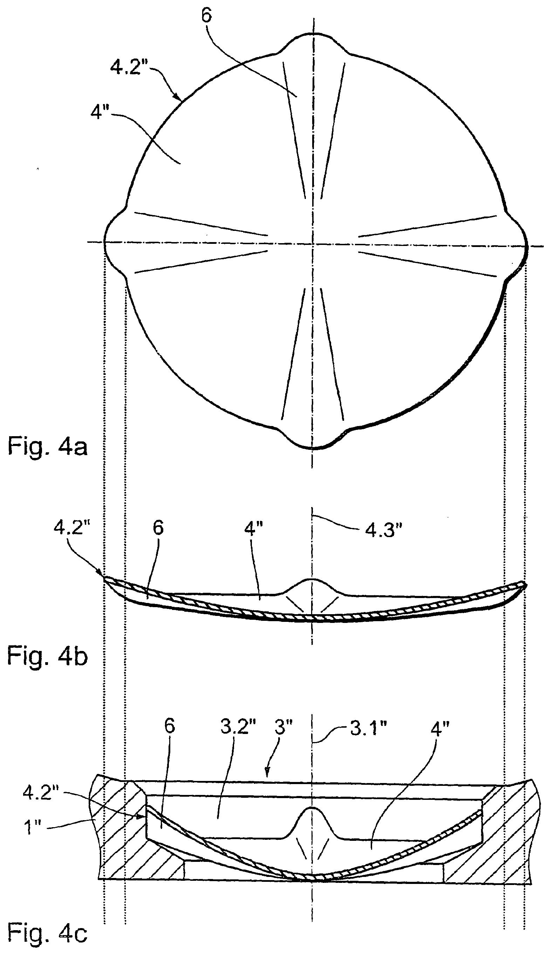

Die Figuren 4a bis 4c zeigen unterschiedliche Teilansichten einer weiteren Ausführungsform eines Deckels 4" des erfindungsgemäßen Endoprothesesystems. So zeigt Figur 4a eine Draufsicht auf den Deckel 4" im Ausgangszustand, Figur 4b eine Schnittansicht des Deckels 4" im Ausgangszustand und Figur 4c eine Schnittansicht des Deckels 4" im in die Durchgangsöffnung 3" einer Hüftpfannenschale 1" eingesetzten Zustand.

Wie schon bei den oben beschriebenen Deckeln weist auch der Deckel 4" quer zu seiner Mittelachse 4.3", die im gezeigten Beispiel im eingesetzten Zustand mit der Längsachse 3.1" der Durchgangsöffnung 3" zusammenfällt, Übermaß gegenüber dem Einsetzabschnitt 3.2" der Durchgangsöffnung 3" auf. Der wesentliche Unterschied zu den oben beschriebenen Deckeln besteht darin, dass der Deckel 4" bereits im Ausgangszustand über seinen Umfang mit vier radial gerichteten Sicken 6 versehen ist. Ein weiterer Unterschied besteht darin, dass der Deckel 4" im Ausgangszustand im Bereich der Sicken 6 eine zur Sickenmitte hin zunehmende, größere Querabmessung als in seinem übrigen Umfangsbereich aufweist. Diese Gestaltung mit der Vorverformung im Ausgangszustand, insbesondere aber der abschnittsweise größeren Querabmessung bewirkt, dass sich die Sicken 6 beim Einsetzen des Deckels 4" in den Einsetzabschnitt 3.2" der Durchgangsöffnung 3" noch stärker ausbilden und so den Deckel 4" weiter stabilisieren. Sie wirken dabei nach Art von Rippen.

Die im Ausgangszustand im Bereich der Sicken 6 zur Sickenmitte hin zunehmende Querabmessung bewirkt nicht nur die entsprechende weitere Ausprägung der Sicken 6 beim Einsetzen in die Durchgangsöffnung 3", sie ist auch so gewählt, dass der Deckelrand 4.2" im eingesetzten Zustand des Deckels 4" im wesentlichen vollständig an der Wandung im Einsetzabschnitt 3.2" der Durchgangsöffnung 3" anliegt und somit die Durchgangsöffnung 3" vollständig verschließt.

Es versteht sich, dass bei anderen entsprechenden Varianten auch eine andere Anzahl von Sicken vorgesehen sein kann. Weiterhin kann vorgesehen sein, dass diese, anders als in den Figuren 4a bis 4c, in einem im Ausgangszustand ansonsten im wesentlichen flachen Deckel ausgebildet sind.

Hinsichtlich der verwendeten Werkstoffe ist anzumerken, dass es sich bei diesen um biokompatible Werkstoffe handelt, die für den jeweiligen Einsatz in dem betreffenden Endoprothesesystem geeignet sind. So werden beispielsweise für die beispielhaft genannten Hüftpfannenschalen in der Regel biokompatible Metalle oder Metalllegierungen ihren Einsatz finden. Für den Deckel werden in der Regel ebenfalls entsprechende Metalle bzw. Metalllegierungen verwendet, da diese hohe Abriebsfestigkeit und gute Verträglichkeit mit dem angrenzenden Knochen besitzen. Es ist aber auch möglich, entsprechende abriebsfeste und knochenverträgliche Kunststoffe für den Deckel einzusetzen. Ebenso ist es möglich, Keramiken mit entsprechendem Verformungspotenzial und entsprechender Körperverträglichkeit zu verwenden.

## Patentansprüche

1. Endoprothesensystem umfassend ein Endoprothesenteil, insbesondere eine Hüftpfannenschale (1; 1'; 1"), zum Befestigen an einem Knochen des menschlichen oder tierischen Körpers mit wenigstens einer Durchgangsöffnung (3; 3'; 3") zum Knochen und einem in einen Einsetzabschnitt (3.2; 3.2'; 3.2") der Durchgangsöffnung (3; 3'; 3") einsetzbaren Deckel (4; 4'; 4") zum im wesentlichen vollständigen Verschließen der Durchgangsöffnung (3; 3'; 3"), wobei der Deckel (4; 4'; 4") zum Erzeugen einer im eingesetzten Zustand quer zur Längsachse (3.1; 3.1'; 3.1") der Durchgangsöffnung (3; 3'; 3") wirkenden Verschlusskraft wenigstens abschnittsweise quer zur Längsachse (3.1; 3.1'; 3,1") der Durchgangsöffnung (3; 3'; 3") Übermaß gegenüber dem Einsetzabschnitt (3.2; 3.2'; 3.2") der Durchgangsöffnung (3; 3'; 3") aufweist, **dadurch gekennzeichnet, dass** der Deckel (4; 4'; 4") als dünnes Plättchen ausgebildet ist, dessen Dicke derart gering und dessen Übermaß gegenüber dem Einsetzabschnitt (3.2; 3.2'; 3.2") der Durchgangsöffnung (3; 3'; 3") derart groß gewählt sind, dass der Deckel (4; 4'; 4") beim unter Aufbringung einer zur Knochenseite gewandten Kraft erfolgenden Einsetzen in die Durchgangsöffnung (3; 3'; 3") eine zur Knochenseite (1.2; 1.2') hin gewölbte Gestalt annimmt.

2. Endoprothesensystem nach Anspruch 1, **dadurch gekennzeichnet, dass der** Deckel als im Ausgangszustand im wesentlichen flaches Plättchen ausgebildet ist.

3. Endoprothesensystem nach Anspruch 1, **dadurch gekennzeichnet, dass** der Deckel (4; 4'; 4") als im Ausgangszustand gewölbtes Plättchen ausgebildet ist.

4. Endoprothesensystem nach einem der vorhergehenden Ansprüche, **dadurch gekennzeichnet, dass** die Dicke des Deckels (4) derart gering und das Übermaß des Deckels gegenüber der Durchgangsöffnung (3) derart groß gewählt sind, dass der Deckel (4) beim Einsetzen in die Durchgangsöffnung plastisch verformt wird.

5. Endoprothesensystem nach einem der vorhergehenden Ansprüche, **dadurch gekennzeichnet, dass** die Dicke und/oder das Übermaß des Deckels (4') gegenüber der Durchgangsöffnung (3') zum Erzielen einer Verschlusskraft gewählt sind, bei der das Endoprothesenteil (1') im Kontaktbereich mit dem in den Einsetzabschnitt (3.2') eingesetzten Deckel (4') zumindest abschnittsweise plastisch verformt wird.

6. Endoprothesensystem nach einem der vorhergehenden Ansprüche, **dadurch gekennzeichnet, dass** im Einsetzabschnitt (3.2) Rastmittel (5) zum Einrasten des Deckelrandes (4.2) in der Durchgangsöffnung (3) vorgesehen sind.

7. Endoprothesensystem nach Anspruch 6, **dadurch gekennzeichnet, dass** die Rastmittel (5) von einem Gewinde im Einsetzabschnitt (3.2) der Durchgangsöffnung (3) gebildet sind.

8. Endoprothesensystem nach Anspruch 7, **dadurch gekennzeichnet, dass** das Gewinde (5) als Standardgewinde, insbesondere als metrisches Gewinde, mit verringerter Gewindetragtiefe ausgebildet ist.

9. Endoprothesensystem nach Anspruch 7 oder 8, **dadurch gekennzeichnet, dass** das Gewinde (5) als Standardgewinde, insbesondere als metrisches Gewinde, mit verringerter Steigung ausgebildet ist.

10. Endoprothesensystem nach einem der vorhergehenden Ansprüche, **dadurch gekennzeichnet, dass** das Endoprothesenteil eine Hüftpfannenschale (1; 1'; 1") ist.

11. Endoprothesenteil für eine Endoprothesensystem nach einem der vorhergehenden Ansprüche mit einer Durchgangsöffnung (3) zum Knochen, die einen Einsetzabschnitt (3.2) für einen Deckel (4) zum Verschließen der Durchgangsöffnung (3) mit Rastmitteln (5) zum Einrasten des Deckelrandes (4.2) aufweist, **dadurch gekennzeichnet, dass** die Rastmittel (5) zum Einrasten des Deckelrandes (4.2) von einem Gewinde gebildet sind.

12. Endoprothesenteil nach Anspruch 11, **dadurch gekennzeichnet, dass** das Gewinde (5) als Standardgewinde, insbesondere als metrisches Gewinde, mit verringerter Gewindetragtiefe ausgebildet ist.

13. Endoprothesenteil nach Anspruch 11 oder 12, **dadurch gekennzeichnet, dass** das Gewinde (5) als Standardgewinde, insbesondere als metrisches Gewinde, mit verringerter Steigung ausgebildet ist

14. Endoprothesenteil nach einem der Ansprüche 11 bis 13, **dadurch gekennzeichnet, dass** es eine Hüftpfannenschale (1) ist.

15. Deckel zum Verschließen einer Durchgangsöffnung (3; 3'; 3") in einem Endoprothesenteil (1; 1'; 1") eines Endoprothesensystems nach einem der vorhergehenden Ansprüche, **dadurch gekennzeichnet, dass** er als gewölbtes Plättchen (4; 4'; 4") ausgebildet ist.
